# EUROPEAN PATENT APPLICATION

(11) **EP 3 928 804 A2**
(43) Date of publication of application: **29.12.2021**
(21) Application number: 20770858.7
(22) Date of filing: 13.03.2020
(51) Int. Cl.: A61L 24/00, A61L 24/08, A61L 27/52, A61L 27/38, C12N 5/00, A61K 9/06, A61K 9/70, A61K 45/06

(54) **HYDROGEL INCLUDING SEROTONIN-MODIFIED HYALURONIC ACID AND USE THEREOF**

(30) Priority: 14.03.2019 KR 20190029214
(71) Applicant: Cellartgen Inc., Seoul 03722 (KR)
(72) Inventor: CHO, Seung Woo, Seoul 06602 (KR); AN, Soo Hwan, Namyangju-si, Gyeonggi-do 12219 (KR)
(74) Representative: Manitz Finsterwald Patent- und Rechtsanwaltspartnerschaft mbB
(86) International application number: PCT/KR2020/003549
(87) International publication number: WO 2020/185041

(57) **Abstract**

The present disclosure relates to a hydrogel comprising hyaluronic acid modified by serotonin, a use thereof, and a method of preparing the same.

## Description

### TECHNICAL FIELD

The present disclosure relates to a hydrogel comprising hyaluronic acid modified by serotonin, a use thereof, and a method of preparing the same.

### BACKGROUND

In surgical operations and treatments, the need for functional Medical materials and devices is increasing, and various types of products have already been developed and used clinically.

Among them, most of the hemostatic agents currently on the market are composed of fibrin-based derivatives, and work in the way of simple clotting by mixing and reacting a fibrinogen protein solution and a thrombin protein solution to form fibrin lumps and physically plugging a bleeding site. In this case, a large amount of hemostatic agent is required to obtain a sufficient hemostatic effect, and the resultant fibrin lumps have the potential to cause side effects that slow skin regeneration or cause adhesion to surrounding tissues.

Further, when a conventional fibrin-based hemostatic agent is used in a situation where the use of a hemostatic agent is needed, tissue adhesion is liable to occur. Thus, an anti-adhesion agent needs to be additionally treated in addition to the hemostatic agent. Such a need for additional treatment results in poor clinical convenience and financial burden.

To overcome the problems of conventional hemostatic agents that physically induce hemostasis, there is an increasing need for a new technology that can improve the hemostatic performance of the hemostatic agents and solve side effects caused by excessive fibrin lumps. Further, there is an increasing need for functional Medical materials and devices capable of performing the above-described complex functions as a single product.

### PRIOR ART DOCUMENT

### [Patent Document]

(Patent Document 1) Korean Patent Laid-open Publication No. 10-2014-0127286
(Patent Document 2) Korean Patent No. 10-1040561

### DISCLOSURE OF THE INVENTION

### PROBLEMS TO BE SOLVED BY THE INVENTION

The present disclosure is conceived to provide a hydrogel including hyaluronic acid modified with serotonin.

The present disclosure is also conceived to provide a hemostatic composition including the hydrogel.

The present disclosure is also conceived to provide a tissue adhesive composition including the hydrogel.

The present disclosure is also conceived to provide a composition for promoting cell differentiation including the hydrogel.

The present disclosure is also conceived to provide a composition for cell culture including the hydrogel.

The present disclosure is also conceived to provide a composition for drug delivery including the hydrogel.

The present disclosure is also conceived to provide a method of preparing the hydrogel.

### MEANS FOR SOLVING THE PROBLEMS

An aspect of the present disclosure relates to a hydrogel including hyaluronic acid modified with serotonin.

In the present disclosure, the term "serotonin (5-hydroxytryptamine)" is a substance derived from tryptophan and found in the brain, internal tissues, platelets, and mast cells, and is also referred to as 5-hydroxytryptamine. The serotonin is mainly found in the gastrointestinal tract, platelets and central nervous system of human and animal and is the molecule that makes you happy and is also referred to as "happiness hormone" even though it is not a hormone. Also, the serotonin has been reported as an important factor in various types of pathological disorders such as psychiatric disorders (depression, aggressiveness, panic attacks, obsessive compulsive disorders, psychosis, schizophrenia, suicidal tendency), neurodegenerative disorders (Alzheimer-type dementia, Parkinsonism, Huntington's chorea), anorexia, bulimia, disorders associated with alcoholism, cerebral vascular accidents, and migraine (Meltzer, Neuropsychopharmacology, 21:106S-115S (1999); Barnes & Sharp, Neuropharmacology, 38:1083-1152 (1999); Glennon, Neurosci. Biobehavioral Rev., 14:35 (1990)).

In the present disclosure, the term "hyaluronic acid" is one of the complex polysaccharides composed of amino acids and uronic acids, and is a polymer compound composed of N-acetylglucosamine and glucuronic acid. The hyaluronic acid is a vivo-derived polymer that hardly causes side effects when applied to a living body and is hydrophilic due to the presence of sugars. Also, the hyaluronic acid retains water and thus keeps your joints physically buffered and lubricated, and is known to be involved in skin flexibility. Further, the hyaluronic acid is one of the major extracellular matrix components in a living body and is degraded in the body and thus is less likely to induce immune response or inhibit tissue regeneration for a long time. Furthermore, the hyaluronic acid is biodegraded by a hyaluronidase and thus can be used as an important material of a drug delivery system by bonding the hyaluronic acid to various drugs.

In the present disclosure, the term "hydrogel" is a gel containing water as a dispersion medium or basic ingredient. In the present disclosure, the hydrogel includes hyaluronic acid modified with serotonin.

Specifically, the hydrogel may be one in which the modified hyaluronic acid is cross-linked by chemical bonding between 5-hydroxyindole moieties of the serotonin, and the cross-linking may be carried out through an oxidation reaction using one or more oxidizing agents selected from the group consisting of hydrogen peroxide (H₂O₂), peroxidase and NaIO₄ (sodium periodate).

Also, properties, such as elasticity and adhesiveness, of the hydrogel are very important factors of the hydrogel which is used as a supporter for treating defects of a specific tissue, e.g., articular cartilage or bone, exposed to higher load, and the properties of the hydrogel of the present disclosure can be regulated by regulating oxidation conditions. Therefore, the hydrogel can be prepared to satisfy conditions.

Specifically, a hyaluronic acid derivative modified with serotonin may have a structure represented by Chemical Formula 1:

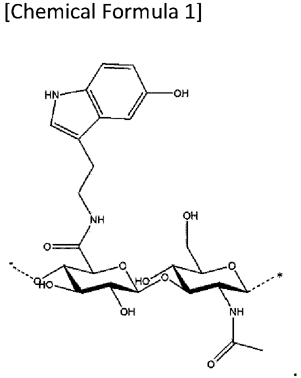

More specifically, the hydrogel may include a repeating unit structure represented by Chemical Formula 1. The repeating unit is not particularly limited, and can be applied by appropriately adjusting the number of repetitions as necessary. For example, if the hydrogel needs to be applied to a broad area, a large number of repeating units may be needed. If the hydrogel is applied to a topical area, the number of repeating units may be relatively small.

Also, specifically, the hydrogel may be used for one or more purposes selected from the group consisting of promotion of blood clotting, hemostasis, inhibition of tissue adhesion, promotion of cell differentiation, cell culture and drug delivery. Further, specifically, the hydrogel may have adhesive properties.

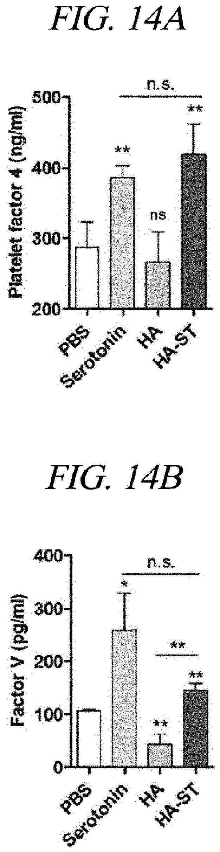

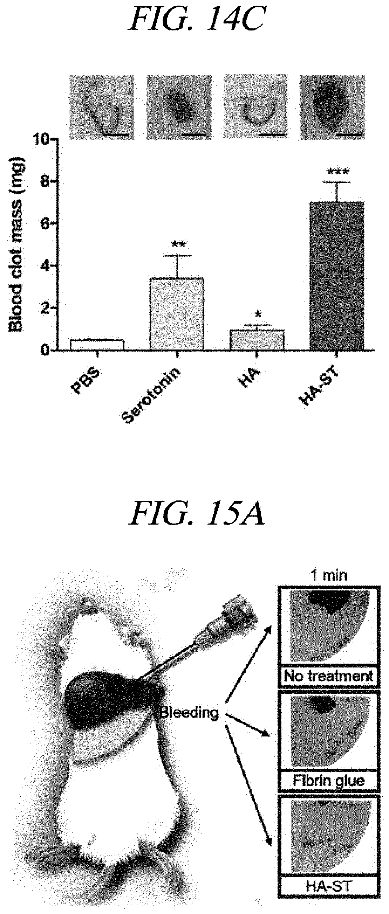

In an example of the present disclosure, it was confirmed that at the time of treatment with the hydrogel of the present disclosure, a secretion amount of blood clotting factor increased and blood clotting occurred in the largest amount compared to the other groups **(****FIG. 14A** to **FIG. 14C****).** Also, it was confirmed that when a serotonin-modified hyaluronic acid hydrogel of the present disclosure or a patch thereof was adhered to a bleeding site of a liver bleeding animal, it exhibited a remarkable hemostatic effect compared to a conventional fibrin-based hemostatic agent **(****FIG. 15A** to **FIG. 15C****,** **FIG. 16A** to **FIG. 16D** and **FIG. 17A to FIG. 17D****).** According to the above result, it was confirmed that the hydrogel of the present disclosure can be used for promotion of blood clotting and/or hemostasis.

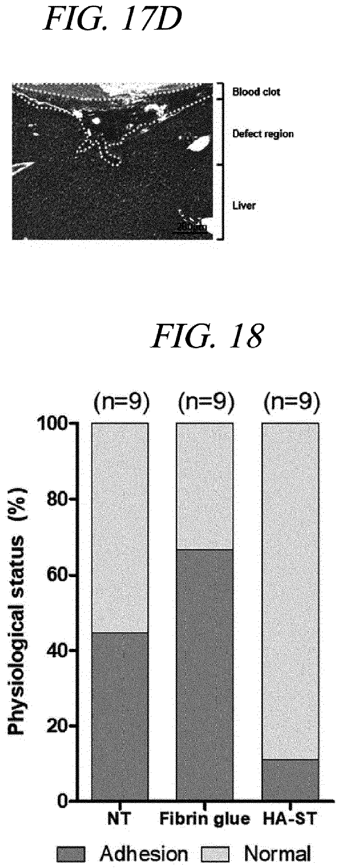

Also, in an example of the present disclosure, it was confirmed that regarding tissue adhesion during hemostasis, a conventional hemostatic agent could not suppress tissue adhesion, whereas tissue adhesion did not occur at the time of treatment with the hydrogel of the present disclosure (**FIG. 18**). Thus, it was confirmed that the hydrogel of the present disclosure can be used for inhibition of tissue adhesion.

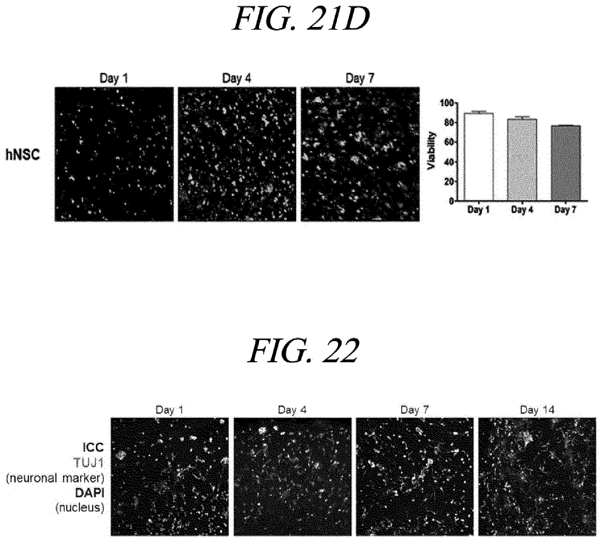

Further, in an example of the present disclosure, human neural stem cells (hNSCs) were encapsulated in the hydrogel of the present disclosure and cultured in three dimensions. As a result, it was confirmed that differentiation of hNSCs was promoted and a neuron-specific markerTuJ1 had a high level of expression and neurites projected well (**FIG. 22**). Thus, it was confirmed that the hydrogel of the present disclosure can be used for promotion of cell differentiation.

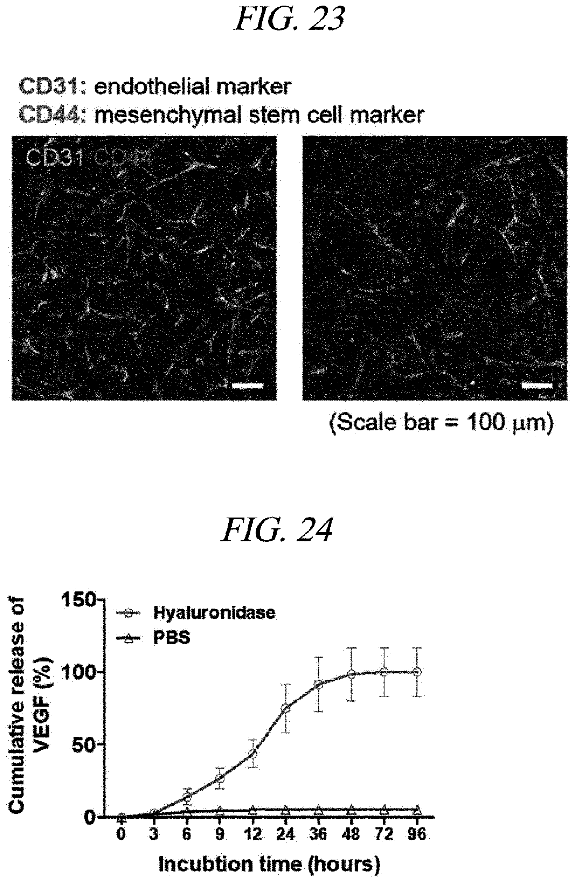

Furthermore, in an example of the present disclosure, human adipose-derived stem cells (hADSCs) and human umbilical vein endothelial cells (HUVECs) were encapsulated in the hydrogel of the present disclosure and cultured in three dimensions. As a result, it was confirmed that each cell proliferated while maintaining its unique morphology **(****FIG. 23****).** Thus, it was confirmed that the hydrogel of the present disclosure can be used for cell culture.

Also, in an example of the present disclosure, it was confirmed that a factor loaded in the hydrogel in an environment where hyaluronic acid can be degraded like an *in vivo* environment was gradually released as the hydrogel was biodegraded **(****FIG. 24****).** Thus, it was confirmed that the hydrogel of the present disclosure can be used for drug delivery.

Also, specifically, the hydrogel may be biodegradable.

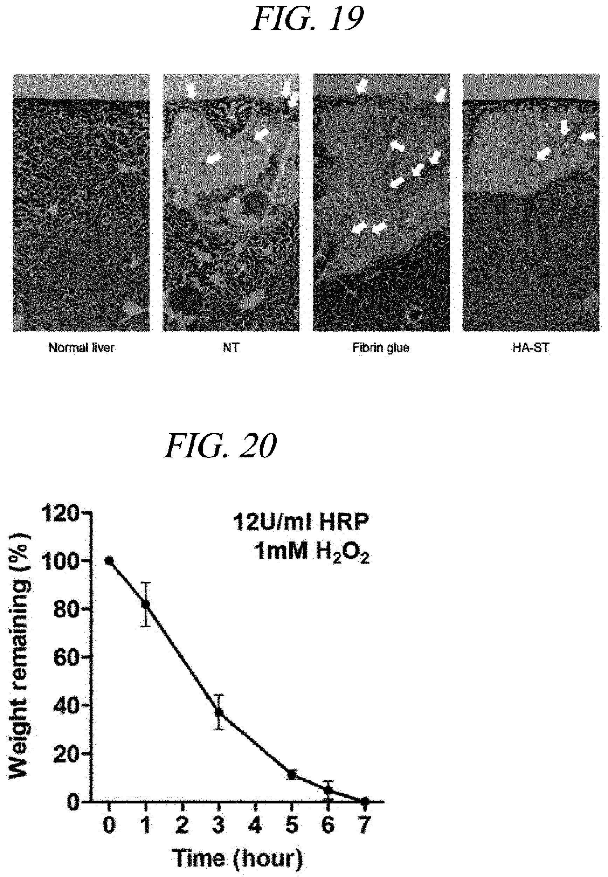

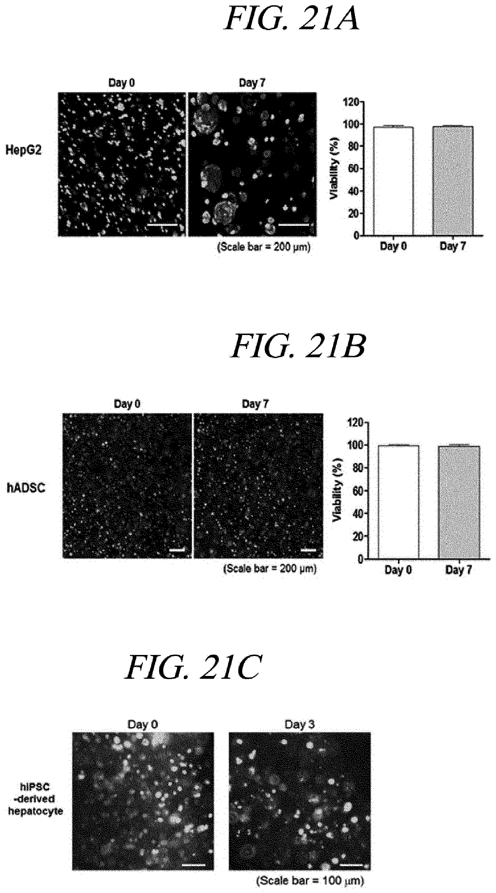

In an example of the present disclosure, it was confirmed that the hydrogel of the present disclosure was gradually degraded in an environment where hyaluronic acid can be degraded like an *in vivo* environment over time **(****FIG. 20****).** Also, the hydrogel of the present disclosure has no cytotoxicity **(****FIG. 21A** to **FIG. 21D****)** and thus is excellent in biocompatibility.

Another aspect of the present disclosure relates to a hemostatic agent composition including the hydrogel. Specifically, the hemostatic agent may have a tissue adhesion inhibiting effect. The hemostatic effect and tissue adhesion inhibiting effect of the present disclosure are the same as described above.

Further, specifically, the hemostatic agent may be in the form of an adhesive patch *(i.e.,* in freeze-dried state) or in the form of a film.

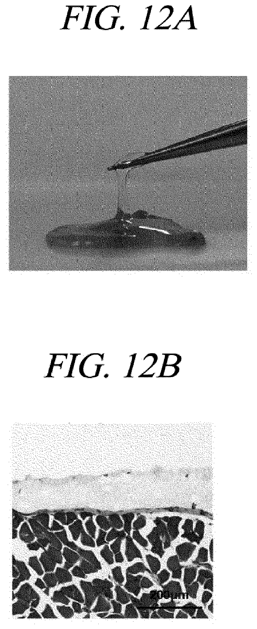

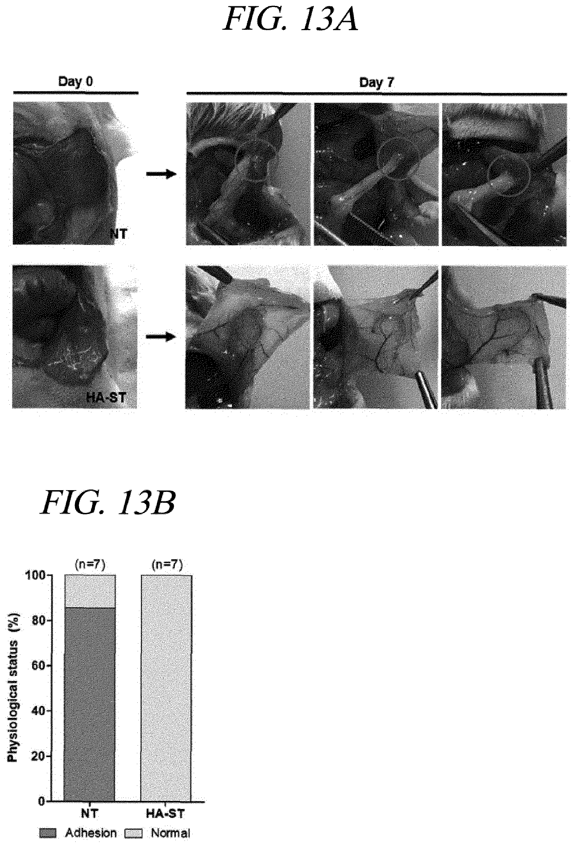

In an example of the present disclosure, it was confirmed that when the hemostatic agent was applied in an adhesive form to an animal model, the hemostatic agent showed an excellent hemostatic effect and tissue adhesion did not occur **(****FIG. 12A** to **FIG. 12B** and **FIG. 13A** to **FIG. 13B****).** Thus, the hemostatic agent can be applied in a form that is adhesive to a surface. The surface may be the surface of any tissue of the body, but is not limited to a specific part.

Yet another aspect of the present disclosure relates to a tissue adhesive composition including the hydrogel. Specifically, the tissue adhesive composition may have a tissue adhesion inhibiting effect. The tissue adhesion inhibiting effect of the present disclosure is the same as described above.

Still another aspect of the present disclosure relates to a composition for promotion of cell differentiation including the hydrogel. Specifically, the cell may be stem cell, fetal stem cell, induced pluripotent stem cell, embryonic stem cell or adult stem cell. The cell differentiation promoting effect of the present disclosure is the same as described above.

Still another aspect of the present disclosure relates to a composition for cell culture including the hydrogel. Specifically, the culture may be a three-dimensional culture.

Also, specifically, cells to be cultured are stem cells, hematopoietic stem cells, hepatic cells, fibrous cells, epithelial cells, mesothelial cells, endothelial cells, muscle cells, nerve cells, immune cells, adipocytes, chondrocytes, bone cells, blood cells or skin cells, but are not limited thereto, and can be applied to all cells capable of growth in the hydrogel of the present disclosure regardless of the cell type. More specifically, the culture may be a simultaneous culture of two or more types of cells.

In an example of the present disclosure, human adipose-derived stem cells (hADSCs) and human umbilical vein endothelial cells (HUVECs) were simultaneously encapsulated in the hydrogel and cultured in three dimensions. As a result, it was confirmed that each cell proliferated while maintaining its unique morphology **(****FIG. 23****).** Thus, the hydrogel of the present disclosure can be used for cell culture and can also be used for complex culture of two or more cell types.

Still another aspect of the present disclosure relates to a composition for drug delivery including the hydrogel.

Specifically, the drug may be encapsulated in the hydrogel, and may be selected from the group consisting of an immune cell activator, an anticancer agent, a therapeutic antibody, an antibiotic, an antibacterial agent, an antiviral agent, an anti-inflammatory agent, a contrast medium, a protein drug, a growth factor, a cytokine, a peptide drug, a hair growth solution and combinations thereof, but is not limited thereto. Any drug that can be encapsulated or loaded in a hydrogel may be applied without limitation.

In an example of the present disclosure, a vascular endothelial growth factor, which is one of growth factors as a drug, was loaded in the hydrogel and a release pattern thereof was checked, and it was confirmed that the growth factor was gradually released as the hydrogel was biodegraded in an environment similar to an *in vivo* environment (**FIG. 24**). Thus, it was confirmed that the hydrogel of the present disclosure can be used for drug delivery. Also, it was confirmed that the drug release can be performed in the form of a delayed release or a slow release.

Further, the drug may be loaded, mounted or encapsulated in the hydrogel of the present disclosure, which may be used in an appropriate form as necessary.

The pharmaceutical composition according to the present disclosure may be prepared into a pharmaceutical dosage form by a well-known method in the art, so that an active component of the composition may be provided via a fast, suspended or prolonged release, after being administered into a mammal. When preparing a dosage form, the pharmaceutical composition according to the present disclosure may further contain a pharmaceutically acceptable carrier, to the extent that this carrier does not inhibit a function of the active component.

The pharmaceutically acceptable carrier may include commonly-used carriers, such as lactose, dextrose, sucrose, sorbitol, mannitol, xylitol, erythritol, maltitol, starch, acacia rubber, alginate, gelatin, calcium phosphate, calcium silicate, cellulose, methyl cellulose, microcrystalline cellulose, polyvinyl pyrrolidone, water, methyl hydroxybenzoate, propyl hydroxybenzoate, talc, magnesium stearate, and mineral oil, but is not limited thereto. In addition, the pharmaceutical composition of the present disclosure may further include a diluent or an excipient, such as fillers, weighting agents, bonding agents, wetting agents, disintegrating agents and surfactants, and other pharmaceutically acceptable additives.

The pharmaceutical composition according to the present disclosure may be administered in a pharmaceutically effective amount. The term "pharmaceutically effective amount" refers to an amount sufficient to prevent or treat a disease at a reasonable benefit/risk ratio applicable to a medical treatment. The effective amount of the pharmaceutical composition of the present disclosure may be determined by a person with ordinary skill in the art according to various factors such as a formulation method, a patient's condition and weight, the patient's gender, age and degree of disease, a drug form, an administration route and period, an excretion rate, reaction sensitivity, etc. The effective amount may vary depending on a route of treatment, a use of excipients and a possibility of being used with other drugs, as recognized by a person with ordinary skill in the art.

The pharmaceutical composition of the present disclosure may be administered to mammals such as mice, livestock, humans, etc. through various routes. Specifically, the pharmaceutical composition of the present disclosure can be administered orally or parenterally (for example, applied or injected intravenously, subcutaneously or intraperitoneally), but may be preferably orally administered. The pharmaceutical composition maybe intravaginally administered to prevent and treat vaginitis. A solid preparation for oral administration may include powder, granule, tablet, capsule, soft capsule, pill, etc. A liquid preparation for oral administration may include suspension, liquid for internal use, emulsion, syrup, aerosol, etc., but may also include various excipients, for example, wetting agent, sweetener, flavoring agent and preservative in addition to generally used simple diluents such as water and liquid paraffin. A preparation for parenteral administration may be used by being formulated into a dosage form of external preparation and sterilized injectable preparation such as sterilized aqueous solution, liquid, water insoluble excipient, suspension, emulsion, eye drop, eye ointment, syrup, suppository and aerosol according to respective conventional methods. Specifically, the pharmaceutical composition may be used in the form of cream, gel, patch, spraying agent, ointment, plaster, lotion, liniment, eye ointment, eye drop, paste, or cataplasma, but is not limited thereto. A preparation for topical administration may be an anhydrous or aqueous form depending on a clinical prescription. As the water insoluble excipient and the suspension, propylene glycol, polyethylene glycol, vegetable oil such as olive oil, and injectable ester like ethyl oleate, etc. may be used. Base materials of the suppository may include witepsol, macrogol, tween 61, cacao butter, laurinum and glycerogelatin.

Specifically, the pharmaceutical composition of the present disclosure may be in the form of an adhesive patch (i.e., in freeze-dried state) or in the form of a film. In an example of the present disclosure, it was confirmed that the pharmaceutical composition of the present disclosure adhered to the tissue and inhibited adhesion between different tissues **(****FIG. 13A** to **FIG. 13B****)** while showing excellent adhesiveness to a surface **(****FIG. 12A** to **FIG. 12B****).** Thus, it can be applied in a form that is adhesive to a surface. The surface includes the skin of any part of the body, the surface inside and outside the tissue, and the like, and is not limited to a specific area.

Still another aspect of the present disclosure relates to a method of preparing a hydrogel including hyaluronic acid modified with serotonin, including: (a) a process of preparing a solution containing hyaluronic acid modified with serotonin by substituting a hydroxyl group (-OH) in hyaluronic acid with serotonin; and (b) a process of cross-linking the modified hyaluronic acid in the solution to form a hydrogel.

Specifically, the process (a) may be a process of substituting a hydroxyl group (-OH) located at R₁ in the hyaluronic acid including a repeating unit structure represented by Chemical Formula 2 with

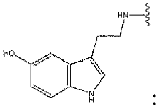

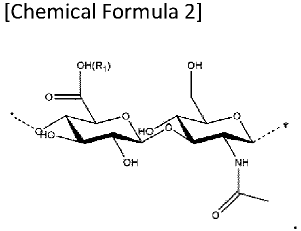

Here, with respect to the solution, the concentration of the modified hyaluronic acid may be 0.1 (w/v)% to 10 (w/v)%.

Further, the cross-linking in the process (b) may be carried out through an oxidation reaction using one or more oxidizing agents selected from the group consisting of hydrogen peroxide (H₂O₂), peroxidase and NaIO₄ (sodium periodate). Here, with respect to the solution, the concentration of the hydrogen peroxide (H₂O₂) may be 0.1 mM to 50 mM, and the concentration of the peroxidase may be 1 U/ml to 100 U/ml (preferably, 10 U/ml to 30 U/ml).

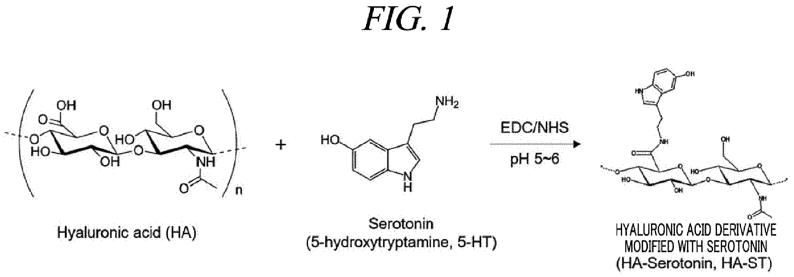

In an example of the present disclosure, a hydrogel including hyaluronic acid modified with serotonin was prepared by introducing the serotonin to the hyaluronic acid and then cross-linking the hyaluronic acid (**FIG. 1**), and the properties, such as elasticity and swelling behavior, of the hydrogel can be regulated by appropriately regulating oxidation conditions in the cross-linking reaction of the as necessary.

Further, the present disclosure provides a hydrogel including hyaluronic acid modified with serotonin to be used in a hemostatic agent composition, a tissue adhesive composition, a composition for promoting cell differentiation, a composition for cell culture or a composition for drug delivery.

Furthermore, the present disclosure provides a hemostasis method, blood coagulation promoting method, tissue adhesion inhibiting method, cell differentiation promoting method, cell culture method or drug delivery method including a process of adhering or administering a hydrogel including hyaluronic acid modified with serotonin to a subject.

In the present disclosure, the term "individual" refers to a subject that needs to be applied with the hydrogel. More specifically, the individual includes human or non-human primates and mammals such as mice, rats, dogs, cats, horses and cows.

### EFFECTS OF THE INVENTION

When the hydrogel including hyaluronic acid modified with serotonin of the present disclosure is used, the degree of cross-linking, cross-linking rate and properties of the hydrogel can be regulated by regulating oxidation conditions.

Also, the hydrogel of the present disclosure exhibits various effects such as hemostasis, promotion of blood clotting, inhibition of tissue adhesion, drug delivery and promotion of cell differentiation. Thus, the hydrogel of the present disclosure can be used for single or multiple purposes. Further, the hydrogel of the present disclosure has very good biocompatibility and is highly applicable because it has little cytotoxicity and can be degraded in the body.

The present disclosure is not limited to the above-mentioned effects, and it should be understood that the present disclosure includes all effects which can be inferred from the constitutions described in the detailed description or the claims.

### BRIEF DESCRIPTION OF THE DRAWINGS

**FIG. 1** is a schematic diagram showing a synthesis process of a serotonin-modified hyaluronic acid derivative.
**FIG. 2** shows the results of ¹H-NMR analysis to check whether serotonin-modified hyaluronic acid is correctly synthesized ((1): a hydrogen proton of a methyl group in a hyaluronic acid skeleton, (2): a hydrogen proton of an aromatic ring of serotonin).
**FIG. 3** shows a chemical structure during a process in which serotonin-modified hyaluronic acid derivatives are cross-linked to form a hydrogel.
**FIG. 4** shows the results of checking cross-linking of a serotonin-modified hyaluronic acid hydrogel by comparing spectra before oxidation and after oxidation through photoelectron analysis (XPS analysis).
**FIG. 5** shows the results of checking cross-linking of serotonin-modified hyaluronic acid hydrogel by comparing spectra before oxidation and after oxidation through Fourier transform infrared spectroscopy (FTIR).
**FIG. 6** shows the results of checking cross-linking of serotonin-modified hyaluronic acid hydrogel through UV-vis (Ultraviolet-visible spectroscopy).
**FIG. 7** shows a serotonin-modified hyaluronic acid hydrogel prepared by a cross-linking method through oxidation.
**FIG. 8** shows the results of comparing the cross-linking rate depending on the oxidation conditions in a process of preparing a serotonin-modified hyaluronic acid hydrogel.
**FIG. 9** shows the results of comparing the elasticity depending on the oxidation conditions in a process of preparing a serotonin-modified hyaluronic acid hydrogel.
**FIG. 10** shows a swelling behavior depending on the oxidation conditions in a process of preparing a serotonin-modified hyaluronic acid hydrogel.
**FIG. 11A** shows the results of measuring a change in adhesiveness of a hydrogel depending on the concentrations of HRP.
**FIG. 11B** shows the results of measuring a change in adhesiveness of a hydrogel depending on the concentrations of hydrogen peroxide.
**FIG. 12A** shows the results of checking the bioadhesiveness of a serotonin-modified hyaluronic acid hydrogel to the tissue (tissue adhesiveness check).
**FIG. 12B** shows the results of checking the bioadhesiveness of a serotonin-modified hyaluronic acid hydrogel to the tissue by H&E tissue staining.
**FIG. 13A** shows the results of checking a tissue adhesion inhibiting effect of a serotonin-modified hyaluronic acid hydrogel (photos of tissue adhesion check).
**FIG. 13B** shows the results of checking a tissue adhesion inhibiting effect of a serotonin-modified hyaluronic acid hydrogel (graph measuring the degree of physical adhesion).
**FIG. 14A** shows the results of checking a blood clotting promoting effect of a serotonin-modified hyaluronic acid hydrogel *in vitro* (graph measuring platelet factor 4).
**FIG. 14B** shows the results of checking a blood clotting promoting effect of a serotonin-modified hyaluronic acid hydrogel *in vitro* (graph measuring factor V).
**FIG. 14C** shows the results of checking a blood clotting promoting effect of a serotonin-modified hyaluronic acid hydrogel *in vitro* (graph measuring the amount of blood clotting in the tissue).
**FIG. 15A** shows the results of checking a hemostatic effect of a serotonin-modified hyaluronic acid hydrogel in a liver bleeding mouse model (experimental schematic diagram).
**FIG. 15B** shows the results of checking a hemostatic effect of a serotonin-modified hyaluronic acid hydrogel in a liver bleeding mouse model (graph measuring the amount of bleeding).
**FIG. 15C** shows the results of checking a hemostatic effect of a serotonin-modified hyaluronic acid hydrogel in a liver bleeding mouse model (measurement of blood clotting time).
**FIG. 16A** shows the results of checking a hemostatic effect of a serotonin-modified hyaluronic acid hydrogel patch in a liver bleeding mouse model (photos of the tissue for hemostatic effect check).
**FIG. 16B** shows the results of checking a hemostatic effect of a serotonin-modified hyaluronic acid hydrogel patch in a liver bleeding mouse model (measurement of blood clotting time).
**FIG. 16C** shows the results of checking a hemostatic effect of a serotonin-modified hyaluronic acid hydrogel patch in a liver bleeding mouse model (graphs measuring the amount of bleeding).
**FIG. 16D** shows the results of checking a hemostatic effect of a serotonin-modified hyaluronic acid hydrogel patch in a liver bleeding mouse model (graphs measuring the amount of bleeding).
**FIG. 17A** shows the results of checking a hemostatic effect of a serotonin-modified hyaluronic acid hydrogel in a liver bleeding mouse model with hemophilia (photos of the tissue for hemostatic effect check).
**FIG. 17B** shows the results of checking a hemostatic effect of a serotonin-modified hyaluronic acid hydrogel in a liver bleeding mouse model with hemophilia (graph measuring the amount of bleeding).
**FIG. 17C** shows the results of checking a hemostatic effect of a serotonin-modified hyaluronic acid hydrogel in a liver bleeding mouse model with hemophilia (measurement of blood clotting time).
**FIG. 17D** shows the results of checking a hemostatic effect of a serotonin-modified hyaluronic acid hydrogel in a liver bleeding mouse model with hemophilia (H&E tissue staining result).
**FIG. 18** shows the results of checking a tissue adhesion inhibiting effect *in vivo.*
**FIG. 19** shows the results of checking the degree of penetration of macrophages at a hemostasis site through toluidine blue staining.
**FIG. 20** shows the results of checking the degree of degradation of a serotonin-modified hyaluronic acid hydrogel over time.
**FIG. 21A** shows the results of confirming that a serotonin-modified hyaluronic acid hydrogel has no cytotoxicity to HepG2 cell.
**FIG. 21B** shows the results of confirming that a serotonin-modified hyaluronic acid hydrogel has no cytotoxicity to hADSC.
**FIG. 21C** shows the results of confirming that a serotonin-modified hyaluronic acid hydrogel has no cytotoxicity to hiPSC-derived hepatocyte.
**FIG. 21D** shows the results of confirming that a serotonin-modified hyaluronic acid hydrogel has no cytotoxicity to hNSC.
**FIG. 22** shows the results of checking a cell differentiation promoting effect of a serotonin-modified hyaluronic acid hydrogel.
**FIG. 23** shows the results of checking a cell culture effect of a serotonin-modified hyaluronic acid hydrogel.
**FIG. 24** shows the results of checking a drug delivery effect and a slow release effect of a serotonin-modified hyaluronic acid hydrogel.

### BEST MODE FOR CARRYING OUT THE INVENTION

Hereafter, the present disclosure will be described in detail with reference to Examples. However, the following Examples are illustrative only for better understanding of the present disclosure but do not limit the present disclosure.

### Example 1. Synthesis of serotonin-modified hyaluronic acid derivative (HA-ST)

To synthesize a serotonin-modified hyaluronic acid derivative (HA-ST), hyaluronic acid having a molecular weight of 200 kDa (Lifecore Biomedical, IL, USA) was dissolved in tertiary distilled water (TDW) to a concentration of 1 mg/ml.

Into the solution, 1-(3-dimethylaminopropyl)-3-ethylcarbodiimide hydrochloride (EDC, Thermo Fisher Scientific, Waltham, MA, USA) and N-hydroxysuccinimide (NHS, Sigma-Aldrich, St. Louis, MO, USA) were added in the same molar ratio as hyaluronic acid, and stirred for 30 minutes at pH 5.5 to 6.0.

Serotonin hydrochloride was added into the solution in a molar ratio of 1:1 with hyaluronic acid, and stirred overnight at room temperature at pH 5.5 to 6.0 to obtain serotonin-modified hyaluronic acid (FIG. 1).

Reaction by-products and unreacted chemicals were removed in a PBS buffer solution of pH 5.0 by using a Cellu Sep T2 dialysis membrane. The product synthesized in the above process was freeze-dried and kept at 4°C.

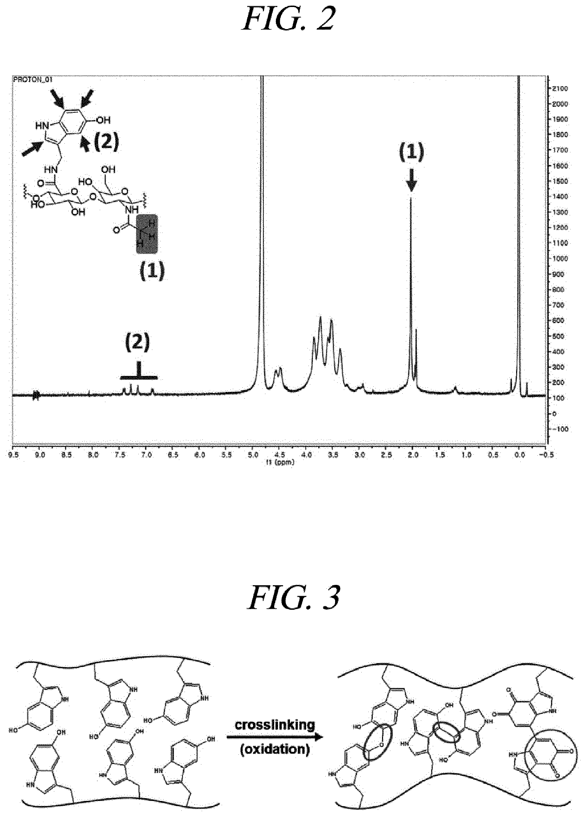

To check whether the serotonin-modified hyaluronic acid is correctly synthesized through the above process, ¹H-NMR (300 MHz, Bruker, Billerica, MA, USA) analysis was performed. A schematic diagram of the synthesis process is as shown in **FIG. 1****.** As a result of the synthesis, it was confirmed that serotonin was bound to hyaluronic acid to have respective peak values as shown in **FIG. 2****.**

### Example 2. Preparation of serotonin-modified hyaluronic acid hydrogel and patch thereof

A hyaluronic acid hydrogel was prepared by cross-linking the serotonin-modified hyaluronic acid derivative of Example 1. Specifically, to this end, a hydrogel was prepared by inducing cross-linking through an oxidation reaction using hydrogen peroxide (H₂O₂) and peroxidase (Horseradish peroxidase, HRP).

As shown in **FIG. 3****,** it was confirmed that the cross-linking of the hyaluronic acid derivative was achieved by chemical bonding between 5-hydroxyindole moieties of serotonin, and the obtained hydrogel had a pale yellow color.

**To** specifically confirm the cross-linking of the hyaluronic acid hydrogel, spectra before oxidation and after oxidation were analyzed through photoelectron analysis (XPS analysis), Fourier transform infrared spectroscopy (FTIR) and UV-vis (Ultraviolet-visible spectroscopy).

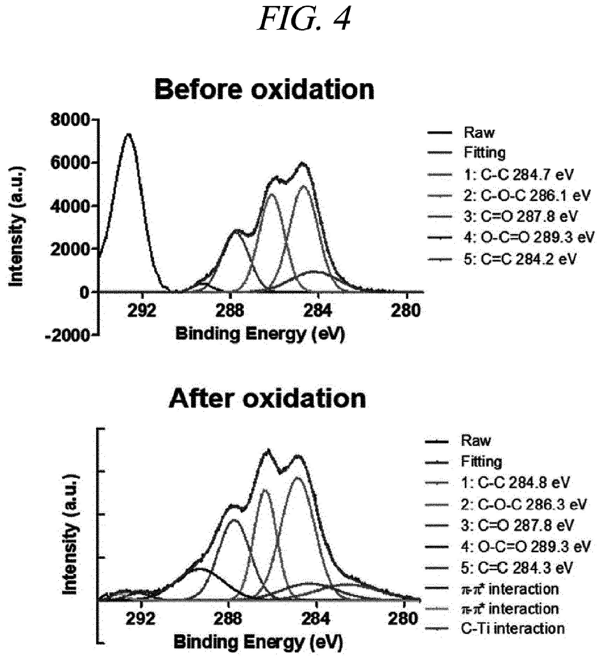

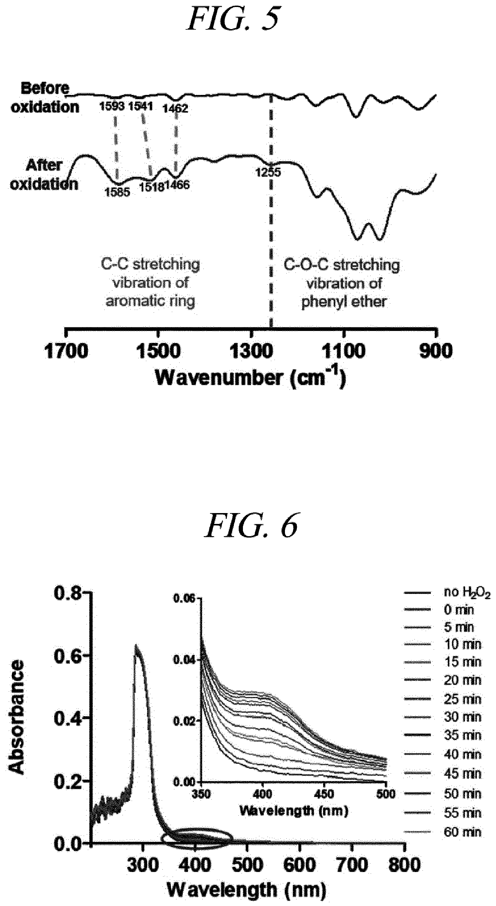

**As** a result, through XPS analysis and FTIR analysis, it was confirmed that the peaks corresponding to C-C bonding and C-O-C bonding, which are bonds generated by the cross-linking reaction, increased **(****FIG. 4** and **FIG. 5****),** and through UV-vis analysis, it was confirmed that a peak (~400 nm) corresponding to quinone increased **(****FIG. 6****).** Thus, it was confirmed that a chemical bond between 5-hydroxyindole moieties of serotonin was generated.

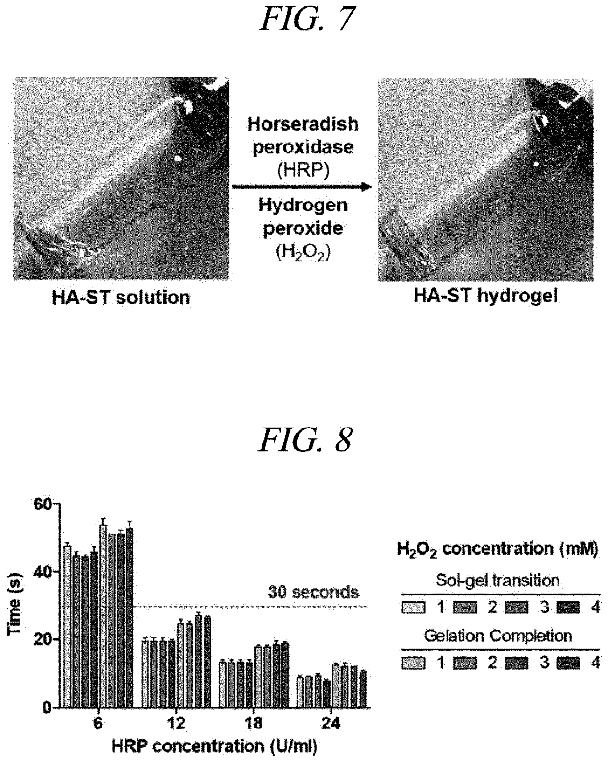

Further, it was confirmed that a serotonin-modified hyaluronic acid hydrogel was prepared by the above-described cross-linking method through oxidation **(****FIG. 7****).**

Also, a 2% serotonin-modified hyaluronic acid solution was mixed with 24 U/ml of HRP and water at a ratio of 8:1:1 and poured in an appropriate volume (3 to 4 ml based on a 35 pie dish) to sufficiently fill the bottom of a petri dish and then frozen and freeze-dried to prepare a patch.

### Example 3. Check of change in cross-linking rate depending on oxidation conditions

In the process of preparing the hydrogel according to Example 2, the concentration of the serotonin-modified hyaluronic acid solution was fixed to 2% by weight and the molar ratio of hydrogen peroxide (H₂O₂) and HRP was changed to measure the gel-sol transition time and gelation completion time of the serotonin-modified hyaluronic acid derivative.

As a result, it was confirmed that the formation rate of the serotonin-modified hyaluronic acid hydrogel was not significantly affected by the concentration of hydrogen peroxide, but was highly dependent on the concentration of HRP.

Specifically, as shown in **FIG. 8****,** when the concentration of HRP was set to 12, 18 and 24 U/ml, the gelation time was less than 30 seconds. However, when the concentration of HRP was set to 6 U/ml, it took 50 seconds to complete gelation, whereas the rate depending on the concentration of hydrogen peroxide did not change much.

### Example 4. Check of change in elasticity of hydrogel depending on oxidation conditions

The viscoelastic modulus of the serotonin-modified hyaluronic acid hydrogel was analyzed by measuring a storage modulus G' and a loss modulus G" stored in a frequency sweep mode in a frequency range of 0.1 to 1 Hz using a rheometer (MCR 102 rheometer, Anton Paar, Ashland, VA, USA). The elasticity of the hydrogel was expressed by calculating the average storage modulus at 1 Hz (n=3).

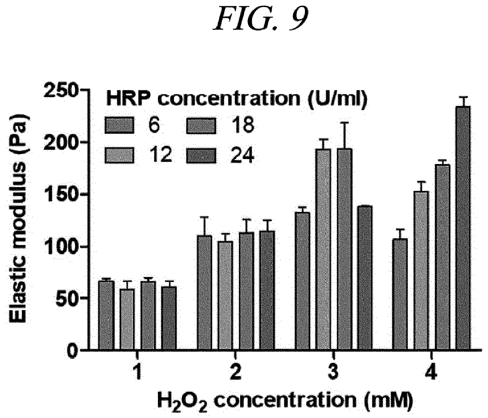

As shown in **FIG. 9****,** the elastic modulus indicating the strength of the hydrogel increased as the concentration of hydrogen peroxide increased in the oxidation condition. Thus, it was confirmed that the strength and elasticity of the hydrogel can be regulated by regulating the concentrations of hydrogen peroxide and HRP.

### Example 5. Check of swelling behavior of hydrogel depending on oxidation conditions

It was confirmed that a swelling behavior was contrary depending on the oxidation conditions. Swelling characteristics were evaluated by measuring the weight of the hydrogel remaining at a specific time point (at days 0, 1, 3, 5 and 7). Specifically, the swelling behavior of the hydrogel formed by varying only the concentration of the serotonin-modified hyaluronic acid solution to 1% by weight or 2% by weight under 1 mM hydrogen peroxide and 12 U/ml HRP conditions was checked.

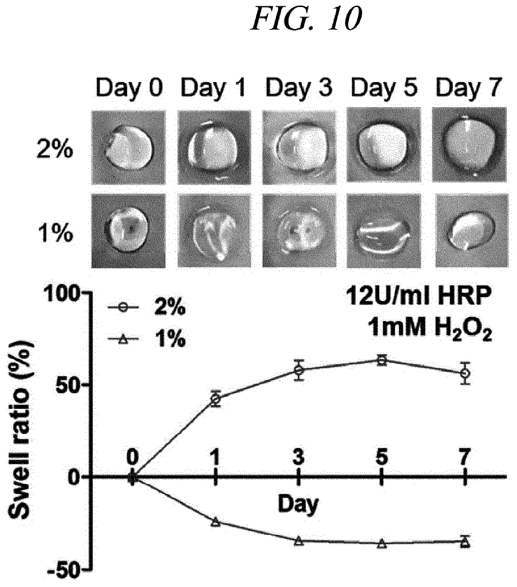

As a result, as shown in **FIG. 10****,** when a serotonin-modified hyaluronic acid solution of 2% by weight was used, the hydrogel swelled, whereas when a serotonin-modified hyaluronic acid solution of 1% by weight was used, the hydrogel shrank. Thus, it was confirmed that the swelling behavior of the hydrogel can be regulated depending on the concentration of the serotonin-modified hyaluronic acid solution.

### Example 6. Analysis on adhesiveness of hydrogel

To evaluate the adhesiveness of the hydrogel prepared in the present disclosure, the hydrogel was cross-linked between a probe and a plate of a rheometer (MCR 102 rheometer, Anton Paar, Ashland, VA, USA), and a force applied thereto was measured while increasing the space between the plates to 10 µm/s (n=3).

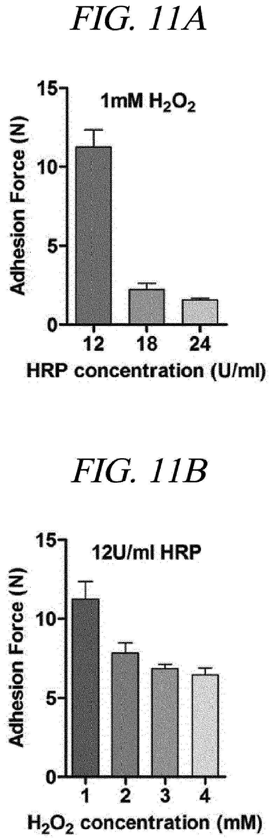

**To** check a difference in adhesiveness depending on the oxidation conditions, the adhesiveness of hydrogels formed by fixing the concentration of hydrogen peroxide to 1 mM and varying only the concentration of HRP to 12, 18 and 24 U/ml was measured. As a result, as shown in **FIG. 11A****,** it was confirmed that the adhesiveness of the hydrogel was highest at the HRP concentration of 12 U/ml under the hydrogen peroxide concentration of 1 mM.

Further, the adhesiveness of hydrogels formed by fixing the concentration of HRP to 12 U/ml and varying the concentration of hydrogen peroxide to 1, 2, 3 and 4 mM was measured. As a result, as shown in **FIG. 11B****,** it was confirmed that the adhesiveness of the hydrogel was highest at the hydrogen peroxide concentration of 1 mM under the HRP concentration of 12 U/ml.

The above results indicate that the adhesiveness of the hydrogel can be regulated by regulating the concentrations of hydrogen peroxide and HRP, and, thus, a desired adhesiveness can be obtained.

### Example 7. Check of adhesiveness of hydrogel to biological tissue

It was checked whether the serotonin-modified hyaluronic acid hydrogel of the present disclosure has a sufficient adhesiveness to the tissue in the body.

Specifically, after the hydrogel was applied to the peritoneal tissue and then pulled to check whether the hydrogel was well adhered to the tissue. As a result, as shown in **FIG. 12A****,** it was confirmed that the hydrogel was well adhered to the tissue with high adhesiveness.

Further, it was checked whether the hydrogel was adhered to the tissue through histological staining (H&E staining) on the peritoneal tissue. As a result, as shown in **FIG. 12B****,** it was confirmed that the serotonin-modified hyaluronic acid hydrogel was firmly adhered to the tissue.

This is because the serotonin-modified hyaluronic acid hydrogel is bound to the protein on the tissue surface due to a chemical reaction that occurs when serotonin molecules are oxidized, so that the serotonin-modified hyaluronic acid hydrogel has tissue adhesiveness. It was confirmed that a hydrogel film can be formed on the tissue surface and the serotonin-modified hyaluronic acid hydrogel can have a sufficient adhesiveness to be adhered to the tissue in the body and maintained well.

### Example 8. Check of tissue adhesion inhibiting effect of hydrogel

It was checked whether the serotonin-modified hyaluronic acid hydrogel of the present disclosure can inhibit abnormal adhesion between tissues. An experiment was conducted using a peritoneal injury mouse model. Specifically, adhesion between the damaged tissue surface and other tissues of the peritoneal injury mouse model was induced, and a difference depending on the presence or absence of treatment with the hydrogel of the present disclosure was checked.

As a result, as shown in **FIG. 13A****,** when adhesion between the damaged tissue surface and other tissues of the peritoneal injury mouse model was induced, abnormal tissue adhesion occurred in almost all animals in the absence of treatment (NT), whereas in the presence of treatment with the serotonin-modified hyaluronic acid hydrogel of the present disclosure (HA-ST), tissue adhesion did not occur in all animals.

Further, the degree of adhesion as described above was also checked quantitatively, and as shown in **FIG. 13B****,** it was also checked numerically that adhesion did not occur at all in the presence of treatment with the serotonin-modified hyaluronic acid hydrogel of the present disclosure.

These results indicate that the hyaluronic acid-based hydrogel thin film formed on the tissue surface has an adhesion inhibiting effect according to the bioadhesion inhibiting mechanism of hyaluronic acid.

### Example 9. Check of blood clotting promoting effect in vitro

To check a hemostatic effect of the serotonin-modified hyaluronic acid hydrogel, ELISA analysis was performed for protein quantification by separating supernatants of samples obtained by mixing and reacting platelet-rich plasma (PRP) isolated from blood with a control PBS, a serotonin solution Serotonin, a hyaluronic acid solution HA and a serotonin-modified hyaluronic acid solution HA-ST, and the amounts of platelet factor 4 and factor V, which are the main blood clotting factors secreted from platelets, were measured.

As a result, as shown in **FIG. 14A****,** in the presence of treatment with the serotonin-modified hyaluronic acid hydrogel, it was confirmed that platelet factor 4 was secreted at a significantly higher level than that of all other groups. Also, as shown in **FIG. 14B****,** in the presence of treatment with the serotonin-modified hyaluronic acid hydrogel, it was confirmed that a secretion amount of factor V was about twice or more compared to that in the presence of treatment only with a hydrogel.

Further, after blood was mixed and reacted with each sample (the control PBS, the serotonin solution Serotonin, the hyaluronic acid solution HA, the serotonin-modified hyaluronic acid solution HA-ST) on a substrate, non-clotted blood was washed off. Then, the weight of the solidified residue was measured. As a result, it was confirmed that blood clotting occurred in the largest amount in the presence of treatment with the serotonin-modified hyaluronic acid hydrogel as shown in FIG. 14C.

The above results indicate that the serotonin-modified hyaluronic acid of the present disclosure promotes blood clotting by promoting serotonin-mediated platelet activity.

### Example 10. Check of hemostatic effect in vivo

To check the hemostatic effect of the serotonin-modified hyaluronic acid hydrogel (and the patch thereof) *in vivo,* liver bleeding models were constructed using 4-week-old female ICR mice (Orient Bio, Seongnam-si). Specifically, after anesthetizing each mouse and dissecting the abdomen, a sterilized filter paper was placed under the liver and bleeding was induced using an 18G needle, and the damaged area was immediately covered with the serotonin-modified hyaluronic acid hydrogel (and the patch thereof) or a fibrin adhesive (TISSEEL, Baxter, Vienna, Austria). Thereafter, the filter paper was replaced every 30 seconds until the observation was completed, and the amount of bleeding was measured by measuring the weight of the collected filter papers. After the evaluation of bleeding was completed, the peritoneum and the incision site were sutured with 6-0 Prolene sutures. An untreated mouse was used as a control. After 7 days of treatment, the mice were sacrificed and their physiological conditions were examined.

As shown in **FIG. 15A****,** a control group NT, a commercial fibrin-based hemostatic agent treatment group Fibrin glue and a serotonin-modified hyaluronic acid hydrogel treatment group HA-ST were prepared with the liver bleeding mouse models. Then, the amount of bleeding was measured.

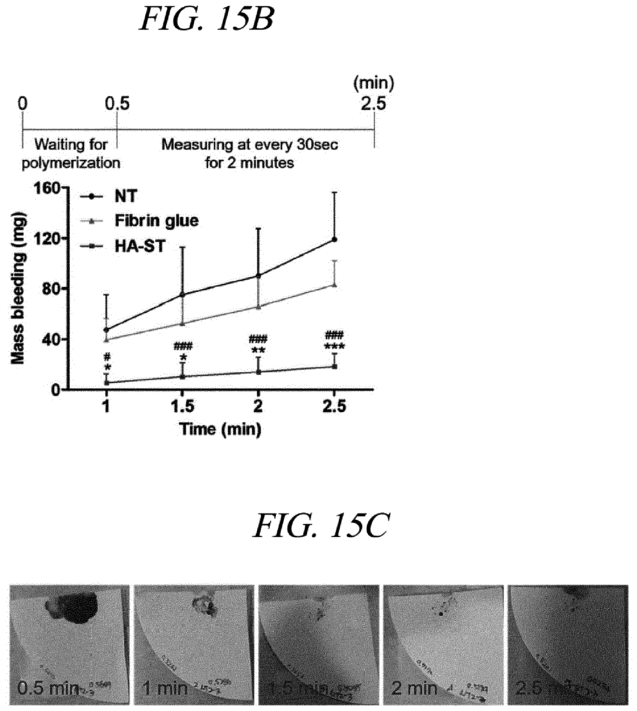

As a result, as shown in **FIG. 15B****,** it was confirmed quantitatively that in the presence of treatment with the serotonin-modified hyaluronic acid hydrogel, the hemostatic effect was remarkably excellent compared to that in the presence of treatment with the conventional commercial fibrin-based hemostatic agent.

Further, photos of the filter paper for blood absorption used to check the amount of bleeding were examined in chronological order, and as a result, it was confirmed that bleeding stopped within a short time in the presence of treatment with the serotonin-modified hyaluronic acid hydrogel as shown in **FIG. 15C****.**

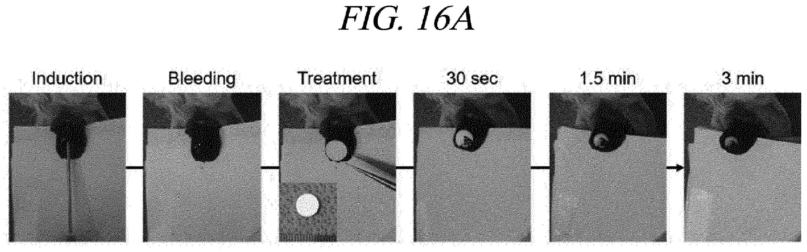

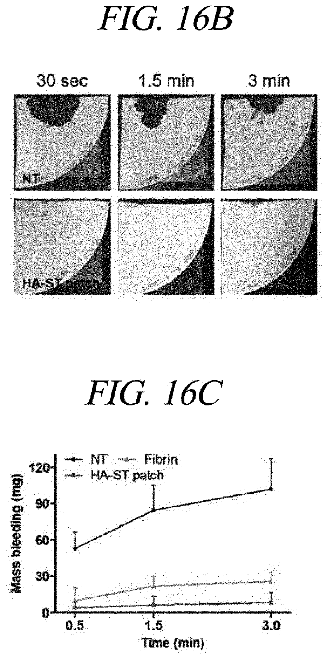

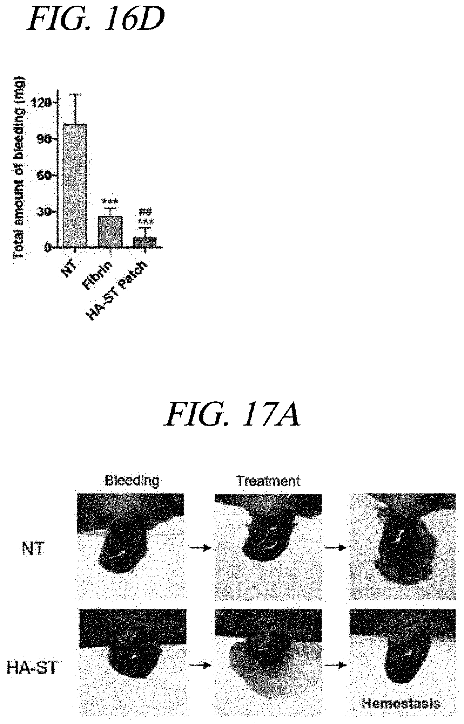

Also, as shown in **FIG. 16A****,** it was confirmed that in the presence of treatment with the serotonin-modified hyaluronic acid hydrogel patch HA-ST Patch, the hemostatic effect was excellent, and as shown in **FIG. 16B****,** bleeding stopped within a significantly short time compared to the control group. Further, it was confirmed quantitatively that as shown in **FIG. 16C** and **FIG. 16D****,** the hemostatic effect was remarkably excellent compared to that in the control group NT or that in the presence of treatment with the conventional commercial fibrin-based hemostatic agent Fibrin.

Furthermore, the hemostatic effect of in a liver bleeding mouse model with hemophilia was checked by constructing liver bleeding models in the same manner as the above method for 6-week-old hemophilic mice (B6;129S-F8^{tm1Kaz}/J, Jackson Laboratory, Bar Harbor, ME, USA).

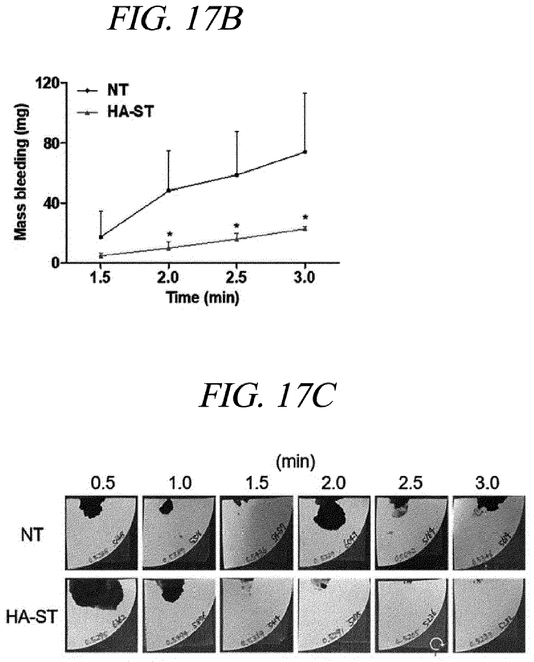

As a result, as shown in **FIG. 17A****,** it was confirmed that even the hemophilic mouse model, which lacks a blood clotting factor and thus cannot stop bleeding well, showed an excellent hemostatic effect compared to the control group, and the amount of bleeding also significantly decreased as shown in **FIG. 17B****.**

Also, as shown in **FIG. 17C****,** it was confirmed that in the presence of treatment with the serotonin-modified hyaluronic acid hydrogel of the present disclosure, bleeding stopped within a significantly short time compared to the control group. Further, as shown in **FIG. 17D****,** it was confirmed through H&E tissue staining that the serotonin-modified hyaluronic acid hemostatic agent induced thrombus formation at a bleeding site to enable effective hemostasis.

### Example 11. Check of tissue adhesion inhibiting effect in vivo

Conventional hemostatic agents have a side effect of causing adhesion to surrounding tissues due to a fibrin mass formed during the hemostatic process. Accordingly, to confirm whether the serotonin-modified hyaluronic acid hemostatic agent of the present disclosure has an adhesion inhibiting effect in addition to the hemostatic action, the degree of tissue adhesion in any three of a total of 9 liver bleeding muse models at days 1, 3 and 7 after treatment with the hemostatic agent was checked.

As a result, it was confirmed that the adhesion inhibiting effect of the serotonin-modified hyaluronic acid hydrogel hemostatic agent was excellent compared to the control group NT not treated with the hemostatic agent and the conventional commercial fibrin hemostatic agent Fibrin glue as shown in **FIG. 18****.**

### Example 12. Check of biocompatibility of hydrogel

In the hemostatic effect experiment, macrophages were stained through toluidine blue staining on liver tissue samples collected at day 3 after treatment with the hemostatic agent to check the degree of penetration of macrophages at a hemostasis site.

As a result, as shown in **FIG. 19****,** the tissue area treated with the serotonin-modified hyaluronic acid hydrogel did not show any particular difference in the degree of penetration of macrophages indicated by the white arrow compared to the normal tissue, which means that no sensitive immune response occurs against the serotonin-modified hyaluronic acid hydrogel of the present disclosure. Thus, it was confirmed that the serotonin-modified hyaluronic acid hydrogel of the present disclosure possesses excellent biocompatibility.

Further, the enzymatic degradation behavior of the serotonin-modified hyaluronic acid hydrogel prepared by mixing a serotonin-modified hyaluronic acid solution of 2% by weight, 1 mM hydrogen peroxide, and 12 U/ml HRP at a volume ratio of 8:1:1 was checked. As a result, as shown in **FIG. 20****,** it was confirmed that the serotonin-modified hyaluronic acid hydrogel of the present disclosure was degraded over several hours by a hyaluronidase. Thus, it was confirmed that the serotonin-modified hyaluronic acid hydrogel of the present disclosure can be degraded in the body. The above-described results indicate that since sensitive immune response did not occur against the hydrogel of the present disclosure and degradation occurred in the body over time, the hydrogel of the present disclosure is excellent in biocompatibility.

### Example 13. Check of cytotoxicity

To evaluate the cytotoxicity and biocompatibility of the serotonin-modified hyaluronic acid hydrogel of the present disclosure, HepG2 cells and human adipose-derived stem cells (hADSCs) (1.0×10⁶ cells /hydrogel 100 µl) were encapsulated in the serotonin-modified hyaluronic acid hydrogel cross-linked under 12 U/ml HRP and 1 mM hydrogen peroxide conditions and subjected to Live/Dead staining while being cultured in three dimensions. Specifically, cell survival was measured at days 0 and 7 with a Live/Dead viability/cytotoxicity kit (Invitrogen, Carlsbad, CA, USA) according to the manufacturer's protocol. The stained cells were observed with a model IX73 fluorescence microscope (Olympus, Tokyo, Japan), and the ratio of viable cells indicated in green to dead cells indicated in red was quantified by manually counting from images (n=3) obtained by the microscope. HepG2 cells were cultured in high glucose Dulbecco's Modified Eagle's medium supplemented with 10% fetal bovine serum (Invitrogen) and 1% penicillin/streptomycin (Invitrogen), and hADSCs were cultured in MesenPRO-RS^{™} medium.

As a result of checking the toxicity, as shown in **FIG. 21A** and **FIG. 21B****,** both HepG2 cells and hADSCs showed a survival rate of about 100% even 7 days later. Thus, it was confirmed that the serotonin-modified hyaluronic acid hydrogel of the present disclosure has little cytotoxicity.

Further, as a result of performing Live/Dead staining as described above while encapsulating hiPSC-derived hepatocytes differentiated from human induced pluripotent stem cells in the serotonin-modified hyaluronic acid hydrogel of the present disclosure and culturing them in three dimensions, few dead cells were observed as shown in **FIG. 21C****.** Thus, it was confirmed that the serotonin-modified hyaluronic acid hydrogel of the present disclosure has no cytotoxicity.

Furthermore, as a result of performing Live/Dead staining as described above while encapsulating human neural stem cells (hNSCs) in the serotonin-modified hyaluronic acid hydrogel of the present disclosure and culturing them in three dimensions, even hNSCs which are difficult to be kept alive showed a survival rate of about 80% even 7 days later. Thus, it was confirmed that the serotonin-modified hyaluronic acid hydrogel of the present disclosure has no cytotoxicity as described above.

### Example 14. Check of cell differentiation promoting effect

Immunostaining (ICC) was performed while encapsulating human neural stem cells (hNSCs) in the serotonin-modified hyaluronic acid hydrogel and culturing them in three dimensions. As a result, as shown in **FIG. 22****,** it was confirmed that a neuron-specific marker TUJ1 had a high level of expression and neurites projected well. This indicates that the serotonin-modified hyaluronic acid hydrogel of the present disclosure is effective in maintaining and differentiating stem cells and can be used as a supporter for three-dimensional culture and transplantation of stem cells.

### Example 15. Check of cell culture effect

Human adipose-derived stem cells (hADSCs) and human umbilical vein endothelial cells (HUVECs) were simultaneously encapsulated in the serotonin-modified hyaluronic acid hydrogel to perform a three-dimensional co-culture, followed by immunostaining.

As a result, as shown in **FIG. 23**, it was confirmed that each cell maintained its unique morphology and induced vascular network formation through interaction with each other, and at the same time, it was confirmed that their corresponding cell markers were normally expressed.

From the above results, it was confirmed that a three-dimensional co-culture of various cells can be performed using the serotonin-modified hyaluronic acid hydrogel of the present disclosure.

### Example 16. Check of drug delivery effect

To check a drug delivery effect of the serotonin-modified hyaluronic acid hydrogel, a vascular endothelial growth factor (VEGF), which is one of growth factors as a drug, was loaded in the serotonin-modified hyaluronic acid hydrogel and then, a release pattern thereof in a hyaluronidase (0.5 U/ml) solution and a PBS solution at 37°C was observed.

As a result, as shown in **FIG. 24**, it was confirmed that the growth factor was hardly released under conditions (PBS) in which the hydrogel was not degraded due to excellent binding ability to protein, but in the presence of the hyaluronidase, which is an environment where hyaluronic acid can be degraded like an *in vivo* environment, the loaded growth factor was gradually released as the hydrogel was biodegraded.

The above results indicate that the serotonin-modified hyaluronic acid hydrogel of the present disclosure can effectively carry a drug and can also control the drug to be gradually released in the body. This means that the serotonin-modified hyaluronic acid hydrogel of the present disclosure has a high applicability as a drug delivery system.

The above description of the present disclosure is provided for the purpose of illustration, and it would be understood by a person with ordinary skill in the art that various changes and modifications may be made without changing technical conception and essential features of the present disclosure. Thus, it is clear that the above-described embodiments are illustrative in all aspects and do not limit the present disclosure. For example, each component described to be of a single type can be implemented in a distributed manner. Likewise, components described to be distributed can be implemented in a combined manner.

The scope of the present disclosure is defined by the following claims rather than by the detailed description of the embodiment. It shall be understood that all modifications and embodiments conceived from the meaning and scope of the claims and their equivalents are included in the scope of the present disclosure.

## Claims

1. A hydrogel including hyaluronic acid modified with serotonin.

2. The hydrogel of Claim 1,
wherein the modified hyaluronic acid includes a repeating unit structure represented by Chemical Formula 1:

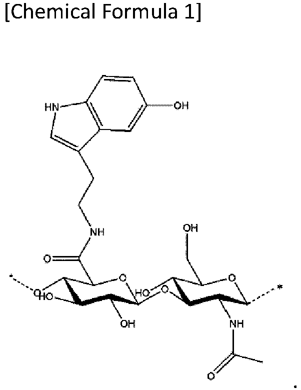

3. The hydrogel of Claim 1,
wherein the hydrogel is one in which the modified hyaluronic acid is cross-linked by chemical bonding between 5-hydroxyindole moieties of the serotonin.

4. The hydrogel of Claim 1,
wherein the hydrogel is adhesive or biodegradable.

5. A hemostatic agent composition including the hydrogel of any one of Claim 1 to Claim 4.

6. The hemostatic agent composition of Claim 5,
wherein the hemostatic agent has a tissue adhesion inhibiting effect.

7. The hemostatic agent composition of Claim 5,
wherein the hemostatic agent is in the form of an adhesive patch or a film.

8. A tissue adhesive composition including the hydrogel of any one of Claim 1 to Claim 4.

9. The tissue adhesive composition of Claim 8,
wherein the tissue adhesive has a tissue adhesion inhibiting effect.

10. A composition for promoting cell differentiation including the hydrogel of any one of Claim 1 to Claim 4.

11. The composition for promoting cell differentiation of Claim 10,
wherein the cell is stem cell, fetal stem cell, induced pluripotent stem cell, embryonic stem cell or adult stem cell.

12. A composition for cell culture including the hydrogel of any one of Claim 1 to Claim 4.

13. The composition for cell culture of Claim 12,
wherein the culture is a three-dimensional culture.

14. A composition for drug delivery including the hydrogel of any one of Claim 1 to Claim 4.

15. The composition for drug delivery of Claim 14,
wherein the drug is encapsulated in the hydrogel.

16. The composition for drug delivery of Claim 14,
wherein the drug is selected from the group consisting of an immune cell activator, an anticancer agent, a therapeutic antibody, an antibiotic, an antibacterial agent, an antiviral agent, an anti-inflammatory agent, a contrast medium, a protein drug, a growth factor, a cytokine, a peptide drug, a hair growth solution and combinations thereof.

17. The composition for drug delivery of Claim 14,
wherein the pharmaceutical composition is in the form of an adhesive patch or a film.

18. A method of preparing a hydrogel including hyaluronic acid modified with serotonin, comprising:
(a) a process of preparing a solution containing hyaluronic acid modified with serotonin by substituting a hydroxyl group (-OH) in hyaluronic acid with serotonin; and
(b) a process of cross-linking the modified hyaluronic acid in the solution to form a hydrogel.

19. The method of preparing a hydrogel of Claim 18,
wherein the process (a) is a process of substituting a hydroxyl group (-OH) located at R₁ in the hyaluronic acid including a repeating unit structure represented by Chemical Formula 2 with

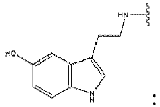

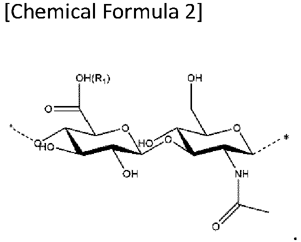

20. The method of preparing a hydrogel of Claim 14,
wherein the cross-linking in the process (b) is carried out through an oxidation reaction using one or more oxidizing agents selected from the group consisting of hydrogen peroxide (H₂O₂), peroxidase and NaIO₄ (sodium periodate).
